# EUROPÄISCHE PATENTANMELDUNG

(11) **EP 1 818 014 A1**
(43) Veröffentlichungstag der Anmeldung: **15.08.2007**
(21) Anmeldenummer: 06101434.6
(22) Anmeldetag: 09.02.2006
(51) Int. Cl.: A61B 5/151

(54) **Testelement mit elastisch gelagerter Lanzette**

(71) Anmelder: F. Hoffmann-la Roche AG, 4070 Basel (CH); Roche Diagnostics GmbH, 68305 Mannheim (DE)
(72) Erfinder: Harttig, Herbert, 67434 Neustadt (DE)
(74) Vertreter: Dick, Alexander

(57) **Zusammenfassung**

Die Erfindung bezieht sich auf ein Verfahren zur Herstellung eines Lanzettensystems (10) und ein Lanzettensystem (10) zum Nachweis eines Analyten in einer Körperflüssigkeit. Das Lanzettensystem (10) umfasst ein Testelement (14) und eine relativ zum Testelement (14) bewegbare Lanzette (26), die am Testelement (14) aufgenommen ist. Die Lanzette (26) ist über eine Elastomerfeder (36) mit dem Testelement (14) verbunden.

## Beschreibung

Die Erfindung bezieht sich auf ein Testelement, insbesondere einen Teststreifen zum Nachweisen von Analyten in einer Körperflüssigkeit, mit einer Lanzette zur Perforation der Haut des Testsubjektes.

### Stand der Technik

Testelemente in Form von Teststreifen zum Nachweis von Analyten im Blut sind seit langem Stand der Technik. Testelemente in Teststreifenform werden insbesondere im Bereich der Blutzuckermessung eingesetzt. Die Messung des Blutzuckerspiegels durch den Patienten selbst hat zu einer ständigen Weiterentwicklung der Testsysteme und der in diesen eingesetzten vorwiegend als Teststreifen ausgebildeten Testelemente geführt. Ziele der Weiterentwicklung waren eine Verbesserung der Analytik, eine Verbesserung der Handhabungssicherheit, die Reduzierung der zu entnehmenden notwendigen Blutvolumina des Patienten und einer Vereinfachung der Handhabung, insbesondere einer Reduzierung der zur Handhabung notwendigen Handhabungsschritte.

Zur Gewinnung des Blutes für die Durchführung der Analyse mittels als Teststreifen ausgebildeter Testelemente sind unterschiedliche Ausführungen von Stechhilfen entwickelt worden, die dazu dienen, die Haut des Patienten zu perforieren und den Austritt der zur Analyse benötigten Blutmenge zu ermöglichen. Hinsichtlich der Verbesserung der Handhabung sind Testelemente vorgeschlagen worden, die eine Kombination von Stechen und Messen ermöglichen.

Aus EP 1 459 683 A1 ist eine Stechhilfe mit Lanzettensystem mit Wiederverwendungsschutz bekannt geworden.

Die Stechhilfe dient zur Erzeugung einer Hautöffnung und umfasst ein Gehäuse, in welches ein Lanzettensystem zumindest zum Teil eingeführt werden kann. Das Gehäuse umfasst des Weiteren ein Halteelement, das mit einem hierzu entsprechenden Halteelement des Lanzettensystems zusammenwirkt, so dass das Lanzettensystem im Gehäuse an einem definierten Ort positioniert werden kann. Das Gehäuse umfasst ferner eine Öffnung, aus der eine Nadelspitze mindestens einer Nadel des Lanzettensystems bei einem Stechvorgang austreten kann. Es ist ein Antriebsmechanismus zum Antreiben der mindestens einen Nadel vorgesehen, so dass die Nadel von einer Ruheposition in eine Stechposition überführt werden kann. Die Nadel ist mit einem Nadelkörper in der Weise verbunden, dass ein Schutzbereich des Nadelkörpers und die Nadel relativ zueinander beweglich sind. In einer ersten Position umgibt der Schutzbereich des Nadelkörpers die Nadelspitze zumindest teilweise, wohingegen in einer zweiten Position der Schutzbereich des Nadelkörpers und die Nadelspitze so zueinander angeordnet sind, dass die Nadelspitze vom Schutzbereich des Nadelkörpers freigegeben wird. Der Nadelkörper beinhaltet des Weiteren einen Sperrmechanismus, der durch ein Zusammenwirken mit der Stechhilfe betätigt wird und den Nadelkörper so verändert, dass nach dem Auswurf des Lanzettensystems aus der Stechhilfe ein Zusammenwirken des Halteelementes der Stechhilfe dem Halteelement des Lanzettensystems bei einem erneuten Wiedereinführen verhindert wird.

Gemäß dieser Lösung ist die metallische Nadel an einem Kunststoffkörper befestigt und mit dem Kunststoffkörper fest verbunden. Am Kunststoffkörper ist ein Bereich ausgebildet, der zur Ankupplung des Lanzettensystems an einen Antriebsstößel vorgesehen ist, so dass die Nadel entlang der Achse in Einstechrichtung bewegt werden kann. Der hintere Bereich des Kunststoffkörpers ist zur Ankupplung an den Antriebsstößel derart ausgebildet, dass zwei Arme, die mit Vorsprüngen versehen sind, die eine formschlüssige Verbindung des Kunststoffkörpers mit der Stechhilfe eingehen. Die Lösung gemäß EP 1 459 683 A1 erfordert mithin eine geräteseitige Kupplung der Lanzette mit einem Stechantrieb.

US 6,561,989 bezieht sich auf einen Teststreifen mit einer aufgesetzten, flach ausgebildeten Lanzette. Die flach ausgebildete Lanzette wird durch eine Flachfeder, die relative beweglich zum Teststreifen ausgeführt ist, beaufschlagt. Die Flachfeder ist in einem Flachmaterial ausgebildet, wobei ein Ende der Feder mit einer Basis verbunden ist und das andere Ende der Feder, das Teil der Lanzette ist, vorspannt. Von Nachteil bei der aus US 6,561,989 B2 bekannten Lösung ist der Umstand, dass die Lanzette relativ breit baut und die Breite des Teststreifens an beiden Seiten erheblich übergreift. Die aus US 6,561,989 B2 bekannte Lösung erfordert relativ Material. Bei der Materialwahl ist zu berücksichtigen, dass der Stahl, aus welchem die Flachfeder gefertigt wird, ein zugelassener Chirurgenstahl sein muss und gleichzeitig gute Federeigenschaften aufweisen sollte. Diese Erfordernisse schließen einander zum Teil aus, so dass hinsichtlich des verwendeten Federstahlmaterials ein Kompromiss geschlossen werden muss.

US 2003/0050573 A1 bezieht sich auf eine Analyse-Einrichtung mit Lanzette und Testelement. Die Lanzette ist nadelförmig ausgebildet und umfasst eine Spitze und einen Lanzettenkörper. Der Lanzettenkörper umgibt die Lanzette zumindest im Bereich ihrer Spitze vollständig. Die Lanzette ist relativ bewegbar zum Lanzettenkörper, wobei der Lanzettenkörper zumindest im Bereich der Spitze der Lanzettennadel aus einem elastischen Material gefertigt ist, in welches die Spitze der Lanzettennadel eingebettet ist. Ein analytisches Testelement ist permanent mit dem Lanzettenkörper verbunden. Während der Stechbewegung tritt die Nadelspitze aus dem elastischen Material aus und wird nach der Durchführung des Einstiches wieder in dieses zurückgezogen. Zur Durchführung einer AnKupplung der Lanzettennadel mit einem Stechantrieb ist das rückwärtige Ende der Lanzettennadel mit einer Verdickung ausgeführt, an der die zu bewegende Lanzettennadel mit einem Stechantrieb gekoppelt wird.

Die Nachteile der Lösung gemäß US 2003/0050573 A1 liegen in der erforderlichen Kupplung zwischen der Lanzettennadel und dem Messgerät, in welches das Lanzettensystem aus Testelement und Lanzettennadel eingelegt wird. Die Kupplung zwischen der Lanzettennadel und dem Stechantrieb ist erforderlich, um die Spitze der Lanzettennadel nach dem Stich wieder aus der Haut in die diese umgebende Einbettmasse zurückzuziehen. Eine solche Kupplung ist geräteseitig vorzusehen und stellt einen erheblichen Aufwand dar. Ferner sind zur Sicherstellung der Kupplung zwischen dem Stechantrieb und der Lanzettennadel Eingriffe an der Lanzettennadel erforderlich. Ferner ist die Kupplung zwischen der Lanzettennadel und dem Stechantrieb stets störungsanfällig. Ein weiterer Nachteil der Lösung gemäß US 2003/0050573 A1 besteht darin, dass die Einbettung der Nadelspitze der Lanzettennadel in ein elastisches Material die Stichbewegung bremst. Dadurch wird die Stichgeschwindigkeit reduziert, was die Schmerzempfindung des Patienten beim Stich erhöht. Um die Lanzettennadel jedoch mit einer das Schmerzempfinden des Patienten schonenden Stichgeschwindigkeit in die Haut einzustechen, ist bei Einbettung der Spitze der Lanzettennadel in das elastische Material der Stechantrieb so zu dimensionieren, dass dieser die mit dem Durchdringen des elastischen Materials verbundene Geschwindigkeitsreduktion kompensiert, d. h. stärker auszulegen ist. Dies wiederum vergrößert den Bauraum des Stechantriebs im Messgerät erheblich.

Die Fertigung einer Kupplung zwischen der Lanzettennadel und dem Stechantrieb stellt einen Zusatzaufwand dar, sowohl hinsichtlich der Lanzettennadel als auch hinsichtlich des Messgerätes.

Die aus dem Stand der Technik bekannten Lösungen hinsichtlich eines kombinierten Testelement/Stechhilfensystems sind insgesamt gesehen entweder mit dem Nachteil einer erhöhten Baugröße verbunden oder erfordern andererseits eine Kupplung zum Messgerät, was einen aus technischer Sicht unbefriedigenden Zustand darstellt.

### Offenbarung der Erfindung

Der Erfindung liegt die Aufgabe zugrunde, ein Testelement/Stechhilfensystem zur Verfügung zu stellen, welches einen einfachen Aufbau aufweist und Kupplungsfrei im Messgerät aufgenommen werden kann.

Erfindungsgemäß wird diese Aufgabe dadurch gelöst, dass an einem im Wesentlichen flach ausgebildeten Testelement, bei dem es sich zum Beispiel um einen Teststreifen handeln kann, auf einer der flachen Seiten des Testelements eine Lanzette oder eine Nadel angeordnet ist, die über ein elastisches Material beweglich mit dem Testelement verbunden ist. Das Testelement umfasst bevorzugt eine Kapillare, die eine nach dem Durchstich der Haut zugängliche Blutprobe von der Haut des Patienten zu einem im Testelement enthaltenen Messbereich transportiert. Bevorzugt befindet sich die Öffnung der Kapillare an der Vorderkante des Testelementes, um die Blutprobe von der Haut des Patienten aufzunehmen.

Auf der einen flach ausgebildeten Seite des Testelementes sind am Testelement ein oder mehrere Messbereiche zum Nachweis des Analyten sowie elektrische Kontakte oder optische Fenster zur messtechnischen Erfassung der Nachweisreaktion aufgebracht. Diese werden beispielsweise im Wege einer Folienbeschichtung der entsprechenden flachen Seite des Testelements auf dieses aufgebracht. Auf der anderen flachen Seite des Testelementes trägt dieses eine Stechhilfe in Form einer Lanzette oder einer Nadel. Anstelle einer Nadel mit rundem oder kreisförmigem Querschnitt kann auch eine Stechhilfe, die einen quadratischen oder einen rechteckförmigen Querschnitt aufweist, eingesetzt werden. Die Lanzette ist parallel zur Längsachse des Testelementes ausgerichtet und befindet sich etwa mittig in Bezug auf die Breite des Testelementes. Das der Kapillare zuweisende Ende der Lanzette ist zu einer Spitze geschliffen oder auf andere Weise, zum Beispiel durch Laserschnitt oder Ätzverfahren, zu einer scharfen Spitze geformt. Es können verschiedenste Profile verwirklicht sein, die zur Herbeiführung einer Hautöffnung geeignet sind. Das der Lanzettenspitze gegenüberliegende, hintere Ende der Lanzette wird stumpf ausgeführt und weist eine Stirnfläche auf

Das erfindungsgemäß vorgeschlagene Lanzettensystem, ein Testelement und eine relativ zu diesem bewegbare Lanzette umfassend, weist eine Verbindung der Lanzette mit dem Testelement aus mindestens einem Strang eines elastischen Materials, bevorzugt eines gummielastischen Materials auf, welcher einerseits mittig an der Lanzette und andererseits auf beiden Längsseiten des bevorzugt streifenförmig ausgebildeten Testelementes befestigt ist. Neben einer strangförmigen Verbindung der Lanzette mit den Längsseiten des Testelementes kann die Verbindung zwischen der Lanzette und dem Testelement auch durch Fäden oder eine Anzahl von Streifen, die sich zwischen den Längsseiten des Testelementes und der mittig auf einer flachen Seite des Testelementes angeordneten Lanzette befinden, ausgebildet sein.

In Ruhestellung befindet sich das spitze Ende der Lanzette innerhalb der Kontur des Testelementes. Das hintere, stumpf ausgebildete Ende der Lanzette kann sowohl innerhalb der Kontur des Testelementes liegen, als auch über die Kontur des Testelementes hinausragen. In einer bevorzugten Ausführungsform ist die Länge der Lanzette so bemessen, dass diese geringer ist als die Länge des Testelementes. An der der Kapillaröffnung abgewandten Hinterkante des Testelementes ist dieses mit einer V-förmigen oder rechtwinklig ausgebildeten Ausnehmung versehen, durch welche der Stechantrieb des Messgerätes das hintere Ende der Lanzette kontaktiert und einen Vorschub der Lanzette relativ zum Testelement bewirkt.

Die Lanzette ist zumindest im Bereich der scharfen Spitze von einer Folientasche umschlossen. Die Folientasche stellt nach der Sterilisation bevorzugt mit β- oder γ-Strahlen die dauerhafte Sterilität der Lanzettenspitze während der Lagerung sicher. Die Folientasche ist bevorzugt fest mit der Außenkante des Testelementes verbunden, so zum Beispiel mit diesem verschweißt. Gemeinsam mit der Folientasche wird auch das elastische Material mit dem Testelement verbunden. Bevorzugt erfolgt die Verbindung der Folientasche mit dem Testelement nach der Sterilisation. Dadurch wird sichergestellt, dass die Nachweischemie zum Nachweis der oder des Analyten der Sterilisation nicht unterworfen und dadurch in ihrer Wirksamkeit nicht beeinträchtigt wird.

Das Testelement trägt für jede Benutzung eine frische ungebrauchte und scharfe Lanzette. Bei Einsatz des erfindungsgemäß vorgeschlagenen Lanzettensystems entfällt die Notwendigkeit, eine separate Stechhilfe mitzuführen und zu benutzen. Durch das scharf geformte Ende der Lanzette wird eine erprobte und zuverlässige, schmerzarme und in Großserie herstellbare Technik zur Erzeugung einer Öffnung in der Haut eines Patienten angewandt. Die Folientasche, in welcher die Lanzette verpackt wird, stellt die Sterilität der Lanzette und insbesondere der Lanzettenspitze sicher.

Durch die erfindungsgemäß vorgeschlagene Lösung ist auch die Handhabung eines Testelementes manuell möglich, wenn kein Messgerät zur Hand ist, ohne Gefahr einer unbeabsichtigten Berührung der frischen oder der benutzten Spitze der Lanzette und ohne Gefahr einer unbeabsichtigten Verletzung. Die elastisch ausgebildete Verbindung zwischen dem Testelement und der Lanzette stellt sicher, dass die Lanzettenspitze nach Durchführung des Stiches wieder in die Kontur des Testelementes zurückgezogen wird, wodurch eine unbeabsichtigte Verletzung ausgeschlossen ist. Der in Form einer Elastomerfeder, bevorzugt aus einem gummielastischen Material gefertigten Verbindung zwischen den Längsseiten des Testelementes und dem Lanzettenkörper steht eine sehr einfache Mechanik für die Bewegung der Lanzette bei Einsatz in einem Messgerät zur Verfügung. Wird das erfindungsgemäß vorgeschlagene Lanzettensystem in einem Messgerät eingesetzt, so kann dieses ohne eine Kupplungsstelle hergestellt werden, da lediglich das stumpfe Ende der Lanzette durch Vorschub eines Stößels, beispielsweise durch den im Messgerät angeordneten Stechantrieb, zu beaufschlagen ist, wodurch die Lanzette aus der Kontur des Testelementes über dessen Vorderkante hinaus zur Durchführung einer Stichbewegung entgegen der Wirkung der Elastomerfeder auszulenken ist. Am Messgerät entfällt die Notwendigkeit einer geräteseitigen Kupplung mit der am Testelement bewegbar aufgenommenen Lanzette. Die Elastomerfeder funktioniert zuverlässiger und störungsunanfälliger als viele mechanische Kupplungen zwischen dem Messgerät und der Lanzette. Ferner kann der Aufwand zur Gestaltung einer lanzettenseitigen Kupplung entfallen. So können zum Beispiel die bei der Lösung gemäß EP 1 459 683 A1 erforderlichen Kunststoffspritzgussteile bei der dort offenbarten zurückholbaren Lanzette entfallen.

Der vom Testelement bewegbar aufgenommenen Lanzette, die von einer Folientasche umschlossen ist, wird bei der Stechbewegung durch das Durchdringen der Folientasche weniger Energie entzogen im Vergleich zu der aus US 2003/0050573 A1 bekannten Lösung, bei der die Lanzette die Einbettmasse vollständig durchfahren muss. Damit wird eine höhere Einstichgeschwindigkeit gewährleistet, was das subjektive Schmerzempfinden des Patienten herabsetzt.

Am Testelement ist auf der der Lanzette abgewandten Seite der eingangs erwähnte Messbereich ausgebildet, zu welchem nach dem Stich eine Blutprobe von der Haut des Patienten durch Kapillarkräfte transportiert wird. Im Messbereich kann sowohl eine optische als auch eine elektrochemische Erfassung des Analyten erfolgen. Das erfindungsgemäß vorgeschlagene Lanzettensystem wird bevorzugt zur Erfassung von klinischen und chemischen Parametern oder auch Koagulationsparametern, wie zum Beispiel Glukose, Lactat, Elektrolyte (z. B. Na⁺, K⁺, Ca⁺⁺), Cholesterin oder Prothrombin unter anderen Parametern eingesetzt.

### Zeichnung

Anhand der Zeichnung wird die Erfindung nachstehend eingehender beschrieben.
Figur 1 zeigt eine Vorderseite des erfindungsgemäß vorgeschlagenen Lanzettensystems, eine elastisch auf einem Testelement aufgenommene Lanzette umfassend,
Figur 2 die Rückseite des Lanzettensystems gemäß der Darstellung in Figur 1,
Figur 3a einen Schnitt durch den Schichtaufbau des Lanzettensystems gemäß Schnittverlauf IIIa-IIIa in Figur 1, etwa auf halber Länge,
Figur 3b einen Schnitt durch den Schichtaufbau des Lanzettensystems gemäß Schnittverlauf IIIb-IIIb in Figur 2, nahe dem vorderen Ende,
Figur 4 die Darstellung des Sterilschutzfolienbandes mit die Ausrichtung ermöglichenden Pilotlöchern,
Figur 5 die Darstellung des Trägerbandes mit beidseitigen Pilotlöchern,
Figur 6 eine alternative Ausführungsvariante der Lanzette und
Figur 6a einen Querschnitt durch die Ausführungsvariante der Lanzette gemäß Figur 6 und
Figur 7 ein zusammenhängendes Band aus Lanzetten.

### Ausführunssbeispiele

Nachfolgend wird unter dem Begriff Lanzettensystem eine Kombination eines Testelementes 14 mit einer daran bewegbar aufgenommenen Stechhilfe, die bevorzugt als Lanzette ausgebildet ist, verstanden. Bei dem Testelement 14 kann es sich um einen Teststreifen handeln, der im Wesentlichen flach ausgeführt ist und so beschaffen ist, dass dieser in ein Messgerät zur Bestimmung eines Analyten in einer menschlichen oder tierischen Körperflüssigkeit eingelegt werden kann. Bei der Lanzette 26 kann es sich sowohl um eine in kreisförmigem Querschnitt ausgebildete Nadel oder um ein Material mit quadratischem oder rechteckförmigem Querschnitt handeln, welches mit einer scharfen Spitze oder einer scharfen Schneide versehen ist, um eine Öffnung in der Haut des Patienten zu erzeugen.

Unter Messgerät wird nachfolgend ein solches verstanden, welches das Lanzettensystem aufnimmt und welches die Analyseergebnisse der in einem Messbereich des Lanzettensystems durchgeführten Analyse einer Körperflüssigkeit anzeigt.

Unter Messbereich wird nachfolgend der Bereich des Lanzettensystems verstanden, innerhalb dessen die entnommene menschliche Körperflüssigkeit durch auf das Testelement des Lanzettensystems aufgebrachte Reagenzien, um ein Beispiel zu nennen, ermittelt wird. Innerhalb des Messbereiches kann die Messung sowohl auf elektrochemischem Wege als auch auf optischem Wege erfolgen.

Unter Folientasche wird nachfolgend eine bevorzugt rechteckig ausgebildete Doppellage einer Kunststofffolie verstanden, die entweder an allen vier Kanten miteinander fest verbunden, bevorzugt verschweißt ist, und wobei im Flächeninneren keine Verbindung zwischen den beiden Lagen der Kunststofffolie besteht oder bei der die Doppellage durch Falten an einer Kante gebildet wird. Zur Ausbildung der Folientasche werden die beiden Lagen der Kunststofffolie an den verbleibenden drei Kanten fest miteinander verbunden, bevorzugt verschweißt.

Als Ruhestellung wird nachfolgende die Position der Lanzette oder Nadel auf dem Testelement verstanden, in der die Nadel oder Lanzette ohne Einwirkung äußerer Kräfte verharrt. In Ruhestellung ist das elastische Material entspannt.

Als Ausformungen werden Ausnehmungen im Bereich der Elastomerfeder verstanden, die durch Formgebung bei der Bildung des elastischen Bandes in flüssigem oder teilflüssigem Zustand des Elastomermaterials gebildet werden.

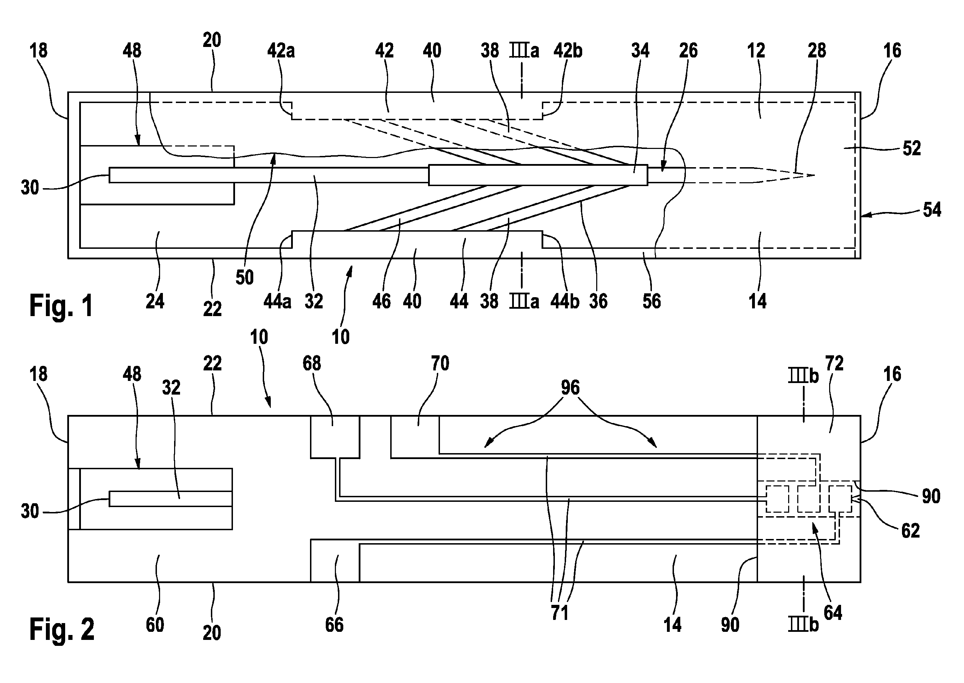

Figur 1 zeigt die Vorderseite des erfindungsgemäß vorgeschlagenen Lanzettensystems, ein Testelement mit darauf elastisch gelagerter Lanzette umfassend.

Der Draufsicht auf eine Vorderseite 12 eines Lanzettensystems 10 ist entnehmbar, dass ein Testelement 14 eine Fläche 24 aufweist, die von einer Vorderkante 16, einer Hinterkante 18, einer 1. Längsseite 20 sowie einer 2. Längsseite 22 begrenzt ist. Entlang der 1. Längsseite 20, der Hinterkante 18 und der 2. Längsseite 22 erstreckt sich eine Umlaufkante 56 um die Fläche 24 des Testelementes 14. Die Umlaufkante 56 umfasst an der 1. Längsseite 20 und der 2. Längsseite 22 des Testelementes 14 einen 1. verbreiterten Bereich 42 sowie an der 2. Längsseite 22 einen 2. verbreiterten Bereich 44. Diese verbreiterten Bereich 42 bzw. 44 stellen jeweils ein Federwiderlager 40 für eine Elastomerfeder 46 dar.

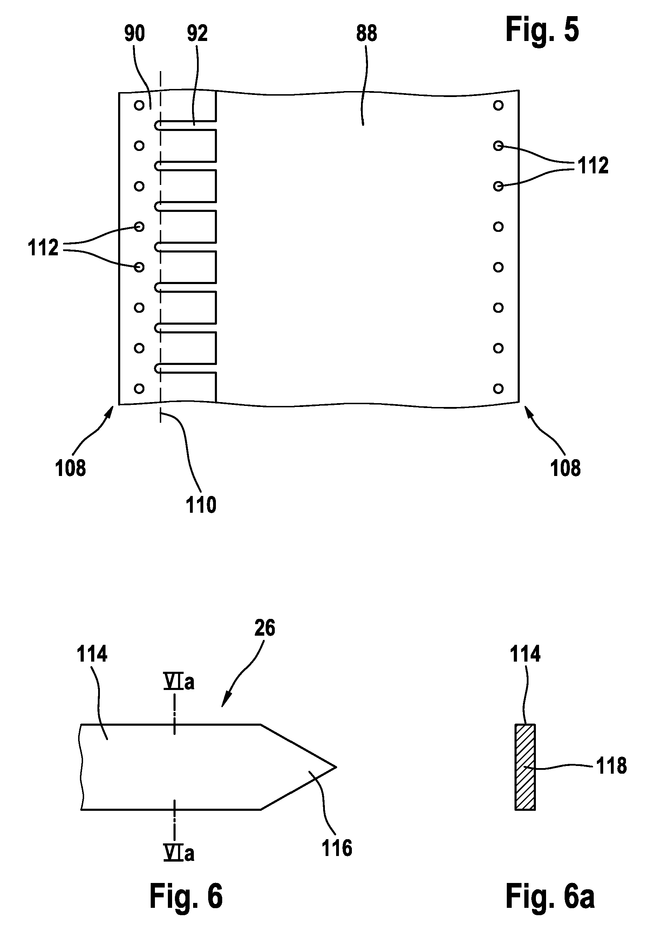

Mittig auf der Fläche 24 des Testelementes 14 ist eine Lanzette 26 angeordnet. In der in Figur 1 dargestellten Ausführungsvariante ist die Lanzette 26 als Nadel ausgebildet und weist demzufolge einen kreisförmigen Querschnitt auf. Anstelle einer nadelförmig ausgebildeten Lanzette 26 ist es selbstverständlich auch möglich, die Lanzette 26 in anderen als nadelförmigen Geometrien auszubilden, vergleiche die in Figuren 6 bzw. 6.1 dargestellten alternativen Ausführungsformen.

Die Lanzette 26 umfasst eine Lanzettenspitze 28, die geschärft ist. Die Lanzettenspitze 28 kann mit einem Anschliff versehen sein, der in den verschiedensten Geometrien ausgeführt sein kann, um beim Einstich der Lanzettenspitze 28 in die Haut eines Testsubjektes eine einen Körperflüssigkeitsaustritt ermöglichende Öffnung in der Haut zu erzeugen. Die Lanzette 26 umfasst ferner einen Lanzettenkörper 32. An dem von der Lanzettenspitze 28 abgewandten Ende ist der Lanzettenkörper 32 in einem stumpfen Ende 30 ausgebildet. Das stumpfe Ende 30 kann zum Beispiel lediglich als Flachfläche ausgebildet werden. Der Lanzettenkörper 32 der Lanzette 26 ist etwa in der Mitte von einer Kunststoffummantelung 34 umschlossen.

Zwischen der Kunststoffummantelung 34 des Lanzettenkörpers 32 und den am 1. verbreiterten Bereich 42 und am 2. verbreiterten Bereich 44 ausgebildeten Federwiderlagern 40 erstreckt sich eine Elastomerfeder 36. Die Elastomerfeder 36 weist auf beiden Seiten der Lanzette 26 mindestens einen Elastomerstrang 38 auf. Die zwischen der Kunststoffummantelung 34 des Lanzettenkörpers 32 und den Federwiderlagern 40 verlaufenden Bereiche der Elastomerfeder 36 können strangförmig, fadenförmig oder auch streifenförmig ausgebildet sein. Die Elastomerfeder 36 wird durch Ausstanzungen, Einschnitte oder Ausformungen 46 in einem thermisch schmelzbarem Elastomer (z. B. Geniomer der Firma Wacker, Burghausen oder Pellethane der Firma Dow Plastics) ausgebildet. Die einzelnen Stränge 38 sind durch die Ausstanzungen, Einschnitte oder Ausformungen 46 in einem Bereich des thermisch schmelzbaren Elastomers ausgebildet, in dem dieses eine reduzierte Dicke von zum Beispiel maximal 200 µm aufweist. Der Darstellung gemäß Figur 1 ist entnehmbar, dass die dort dargestellte Elastomerfeder 36 beiderseits der Kunststoffummantelung 34 des Lanzettenkörpers 32 jeweils zwei Einzelstränge 38 aufweist, durch welche die Kunststoffummantelung 34 des Lanzettenkörpers 26 mit dem 1. verbreiterten Bereich 42 und dem 2. verbreiterten Bereich 44 der Umlaufkante 56 elastisch verbunden ist.

Im Bereich der Hinterkante 18 des Lanzettensystems 10 ist eine Ausnehmungskontur 48 dargestellt, in welcher optional beim Einlegen des Lanzettensystems 10 in ein Messgerät die Umlaufkante 56 an der Hinterkante 18 und die Fläche 24 des Testelementes 14 eingeschnitten werden kann, um das stumpfe Ende 30 der Lanzette 26 freizulegen. Wenngleich die Ausnehmungskontur 48 in der Darstellung gemäß Figur 1 rechteckförmig dargestellt ist, kann diese auch halbkreisförmig oder V-förmig ausgebildet sein. Das Einschneiden des Lanzettensystems 10 entlang der Ausnehmungskontur 48 im Bereich der Hinterkante 18 des Lanzettensystems 10 ist jedoch nicht zwingend erforderlich. Das Durchtrennen des Materials des Lanzettensystems 10 entlang der Ausnehmungskontur 48 kann während des Einlegens des Lanzettensystems 10 in ein Messgerät erfolgen, manuell herbeigeführt werden oder auch vollständig entfallen.

Das in Figur 1 von der Vorderseite dargestellte Lanzettensystem 10 umfasst ein Sterilschutz-Folienband, welches eine Folientasche 50 bildet und die Lanzette 26 und die Elastomerfeder 36 umschließt. Die Unterseite 82 der Folientasche 50 wird mit dem Testelement 14 bevorzugt entlang von dessen Umlaufkante 56 fest verbunden, bevorzugt verschweißt. Entlang der Vorderseite 16 des Lanzettensystems 10 ist die Folientasche 50 gefaltet; ein vor der Lanzettenspitze 28 liegender Falz ist durch Bezugszeichen 54 bezeichnet. Die Oberseite 52 der Folientasche 50 ist entlang der Umlaufkante 56 mit der Unterseite 82 fest verbunden, bevorzugt verschweißt. Im Bereich der verbreiterten Bereiche 42 und 44 wird die Oberseite 52 der Folientasche 50 mit der Oberseite der verbreiterten Bereiche 42 bzw. 44 und die Unterseite 82 der Folientasche 50 mit der Unterseite der verbreiterten Bereiche 42, 44 fest verbunden. Schmalkanten 42a, 42b, 44a und 44b der verbreiterten Bereiche 42, 44 sind abgeflacht und gehen gas- und keimdicht in die Verbindung der Oberseite 52 und der Unterseite 82 der Folientasche 50 über.

Der Darstellung gemäß Figur 2 ist eine Draufsicht auf die Rückseite des in Figur 1 von der Vorderseite her dargestellten erfindungsgemäßen Lanzettensystems zu entnehmen.

Aus der Darstellung gemäß Figur 2 geht hervor, dass auf einer Rückseite 60 des Lanzettensystems 10 eine Folie mit Elektrodenstruktur 96 aufgebracht ist. Bei der Folie mit Elektrodenstruktur 96 handelt es sich bevorzugt um eine aus einem elektrisch leitenden Material wie zum Beispiel Gold gefertigte Folie, in der Leiterbahnen 71 zwischen einem Messbereich 64 auf der Rückseite 60 des Testelementes 14 verlaufen und den Messbereich 64 mit einer 1. Elektrode 66 an der 1. Längsseite 20 und mit zwei weiteren Elektroden 68 und 70 an der 2. Längsseite 22 des Testelementes 14 verbinden.

An der Vorderkante 16 des Testelementes 14 ist ein Kapillareingang 62 einer Kapillare 92 dargestellt, über welche zum Beispiel aus einer durch die Lanzettenspitze 28 in der Haut eines Testsubjektes erzeugte Öffnung austretende Körperflüssigkeit, wie zum Beispiel Blut, in den Messbereich 64 gelangt. Im Messbereich 64 kann zum Beispiel auf elektrochemischem Wege der Nachweis eines Analyten in der Körperflüssigkeit erfolgen. Neben einem elektrochemischen Nachweisverfahren kann im Messbereich 64 auch ein optisches Nachweisverfahren zur Bestimmung eines Analyten in einer Körperflüssigkeit des Testsubjektes ablaufen. In diesem Falle werden anstelle der Folie mit Elektrodenstruktur 96 innerhalb des Messbereiches 64 optische Anzeigen, so zum Beispiel optische Fenster, ausgebildet und die Elektrodenstruktur 96 könnte entfallen. Der Messbereich 64 auf der Rückseite 60 des Testelementes 14 ist bevorzugt durch eine Deckelfolie 72 verschlossen. Eine Spacerschicht 90 überdeckt die Leiterbahnen 71 zu den Elektroden 66, 68 und 70.

An der Hinterkante 18 der Rückseite 60 des Testelementes 14 gemäß der Darstellung in Figur 2 ist das Material der Fläche 24 des Testelementes 14 entlang der in Figur 1 dargestellten Ausnehmungskontur 48 entfernt. Dadurch ist in Figur 2 der rückwärtige Bereich des Lanzettenkörpers 32 sowie Teile der Umlaufkante 56 sichtbar. Wird das Material des Testelementes 14 im Bereich der Ausnehmungskontur 48, wie in Figur 2 dargestellt, entfernt, so kann die Energie eines Stechantriebes zur Ermöglichung einer schnellen Stichbewegung unvermindert am stumpfen Ende 30 auf den Lanzettenkörper 32 der Lanzette 26 aufgebracht werden.

Die Funktionsweise des erfindungsgemäß vorgeschlagenen Lanzettensystems 10 stellt sich wie folgt dar:

Das Lanzettensystem 10 wird manuell oder automatisch in ein Messgerät in die Messposition gebracht. Beim Einsetzen des Lanzettensystems 10 kann am hinteren Ende des Lanzettensystems 10 die dieses umgebende Folientasche beidseitig der Lanzette 26 im Bereich der Ausnehmungskontur 48 eingeschnitten werden. Das Einschneiden kann auch später im Messgerät durch den dort vorhandenen Stechantrieb erfolgen oder vollständig entfallen. Die Vorderkante 16 des Lanzettensystems 10 nimmt dabei bevorzugt eine solche Position ein, dass ein Benutzer die Vorderkante 16 mit der Haut, zum Beispiel eines Fingers, in Kontakt bringen kann. Danach erfolgt das Spannen des im Messgerät vorhandenen Stechantriebes. Das Lanzettensystem 10 wird mit seiner Vorderkante 16 mit der Haut in Kontakt gebracht und ein Auslöser für den im Messgerät implementierten Stechantrieb aktiviert. Das Auslösen des Stechantriebes kann auch durch einen ausreichend kräftigen Andruck, zum Beispiel des Fingers, an die Vorderkante 16 des Lanzettensystems 10 erfolgen. Durch Aktivierung des Auslösers wird der Stechantrieb des Messgerätes in Bewegung gesetzt. Dabei schiebt ein zum Beispiel als Stößel ausgebildetes Vorschubelement des Stechantriebes die Lanzette 26 nach vorne in Richtung auf die Vorderkante 16 des Lanzettensystems 10. Auf ihrem Weg nach vorne durchschneidet die Lanzettenspitze 28 der Lanzette 26 zunächst die die Lanzette 26 umgebende Folientasche 50 am Falz 54 und dringt dann in die Haut des Testsubjektes ein. Die Eindringtiefe der Lanzettenspitze 28 der Lanzette 26 in die Haut, wird durch den Vorschub, zum Beispiel eines Stößels, des Stechantriebes bestimmt. Die Stechtiefe kann am Messgerät voreingestellt werden, was durch eine Einstellung des Vorschubweges, zum Beispiel eines Stößels erfolgen kann.

Mit der Vorwärtsbewegung der Lanzette 26 relativ zum Testelement 14, wird auch der mindestens eine elastische Strang 38 der die Lanzette 26 elastisch am Testelement 14 fixierenden Elastomerfeder 36 vorgespannt. Bei Erreichen des Endes der Vorschubbewegung des Stechantriebes fährt dieser wieder in seine Ausgangslage zurück. Die Lanzette 26 wird hingegen durch den mindestens einen gedehnten elastischen Strang 38 der Elastomerfeder 36 wieder in die bei der Stichbewegung bereits durchstochene Folientasche 50 zurückgezogen.

Durch den Stich in die Haut tritt ein Blutstropfen aus. Die Öffnung der Kapillare 62 wird entweder automatisch oder manuell in Kontakt mit dem Blutstropfen gebracht. Das austretende Blut wird durch die Kapillare 92 aufgesaugt und benetzt den Messbereich 64 auf der Rückseite 60 des Testelementes 14 des Lanzettensystems 10. Die Messung der Analytkonzentration erfolgt innerhalb des Messbereiches 64 in an sich bekannter Weise. Nach Beendigung der Messung wird das gebrauchte Lanzettensystem 10 ausgeworfen oder in ein Magazin zur Aufnahme verbrauchter Lanzettensysteme 10 überführt.

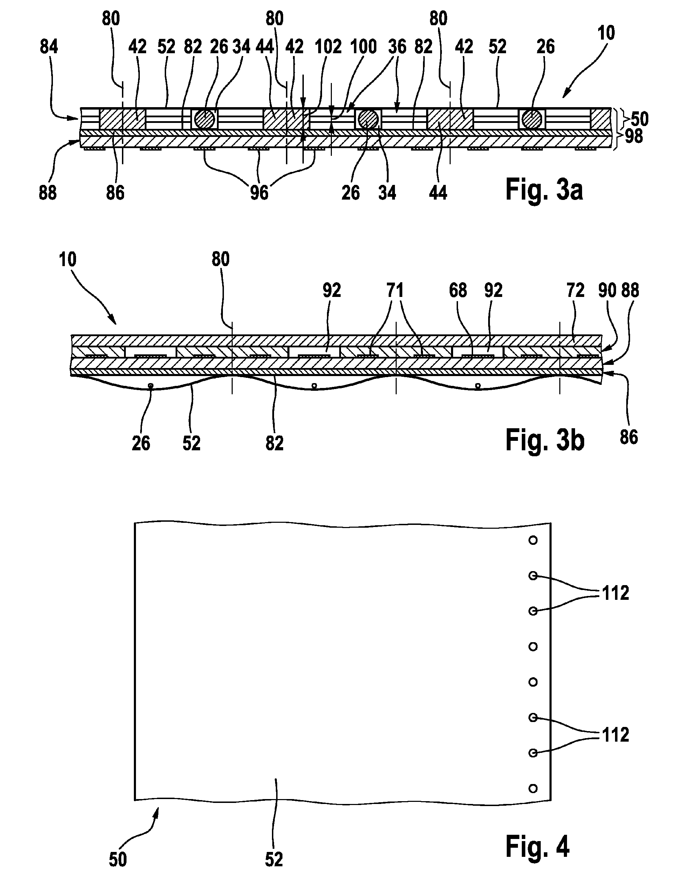

Den Darstellungen gemäß der Figuren 3a und 3b sind Schnitte durch das erfindungsgemäß vorgeschlagene Lanzettensystem, welches in Figur 1 von der Vorderseite her und in Figur 2 von der Rückseite her dargestellt ist, zu entnehmen.

Aus der Schnittdarstellung gemäß Figur 3a geht hervor, dass das Lanzettensystem 10 einen Schichtaufbau 98 aufweist.

Aus dem in Figur 3a dargestellten Schichtaufbau 98 geht hervor, dass das Lanzettensystem 10 als kontinuierliches Band gefertigt werden kann, welches an seiner Oberseite ein kontinuierliches Band 84 mit voneinander beabstandeten, jeweils von der Kunststoffummantelung 34 umgebenen Lanzetten 26 aufweist. Eine Vielzahl von Lanzetten 26 werden in gegenseitigem Abstand von einer Testelementbreite mit einem thermisch schmelzbaren Elastomer (so zum Beispiel Geniomer der Firma Wacker, Burghausen) zu einem kontinuierlichen Band 84 verbunden. Dieses kontinuierliche Band 84 mit voneinander beabstandeten Lanzetten 26 ist von der Folientasche 50 beidseits umschlossen. In Figur 3 ist die Oberseite der Folientasche 50 durch Bezugszeichen 52 gekennzeichnet, die Unterseite der Folientasche 50 ist durch Bezugszeichen 82 kenntlich gemacht. Durch die Folientasche 50, die das kontinuierliche Band 84 mit den einzelnen, durch das elastische Elastomer miteinander verbundenen Lanzetten 26 verbindet, wird durch die Folientasche 50 versiegelt. Aus der Darstellung gemäß Figur 3a geht hervor, dass die Kunststoffummantelung 34, die ihrerseits die einzelnen Lanzetten 26 umschließt, über die einzelnen Stränge 38 der Elastomerfeder 36 mit dem 1. verbreiterten Bereich 42 und dem 2. verbreiterten Bereich 44 verbunden ist. Der 1. verbreiterte Bereich 42 bzw. der 2. verbreiterte Bereich 44 des Lanzettensystems 10 weist eine durch Bezugszeichen 102 gekennzeichnete Dicke auf, die etwa 1/4 bis 5/4 der Dicke der Lanzette 26 entspricht. Die Elastomerfeder 36 ist im thermisch schmelzbaren Elastomer in einer Dicke 100 ausgebildet, die maximal 200 µm beträgt. Die Kunststoffummantelung 34 erstreckt sich entlang der Länge der Lanzette 26 über mindestens 1/10 ihrer Länge. Die verbreiterten 1. und 2. Bereiche 42, 44 des kontinuierlichen Bandes 84 erstrecken sich in einer Breite zwischen 1/10 und 1/4 der Breite der Testelemente 14 und weisen eine Länge von mindestens 1/10 der Länge der Lanzette 26 auf. Die in Figur 3a angedeutete Elastomerfeder 36 (vergleiche Darstellung in Figur 1) wird durch Ausstanzen oder Einschneiden oder Ausformen innerhalb der in der Dicke 100 ausgebildeten Bereiche des kontinuierlichen Bandes 84 erzeugt. Das kontinuierliche Band 84 ist mittels einer Kleberschicht 86, zum Beispiel einem Schmelzkleber, auf ein Folienband 88 geklebt, welches als Trägerband dient. In der Darstellung gemäß Figur 3b gemäß Schnittverlauf IIIb-IIIb in Figur 2 erstreckt sich oberhalb des Folienbandes 88 eine doppelseitig klebende Spacerschicht 90, in welcher Kapillarkanäle 92 ausgebildet sind, über welche die an der Stichstelle entnommene Körperflüssigkeit zu dem in Figur 3b nicht dargestellten Messbereich 64 des Lanzettensystems 10 transportiert wird. An der Unterseite des Folienbandes 88 befindet sich die in Figur 2 auf der Rückseite 60 des Lanzettensystems 10 dargestellte Folie mit Elektrodenstruktur 96. Die Kapillarkanäle 92 bzw. die Spacerschicht 90 werden von der Deckelfolie 72 überdeckt, die in Figur 2 teilweise dargestellt ist und dort den Messbereich 64 des Lanzettensystems 10 überdeckt. Bezugszeichen 52 bezeichnet die Oberseite der Folientasche 50 aus Sterilschutz-Folienband.

In der Darstellung gemäß Figur 3a sind die innerhalb des kontinuierlichen Bandes 84 miteinander verbundenen Lanzetten 26 als Nadeln ausgeführt. Die Lanzetten 26 können aus einem Edelstahl mit einem Durchmesser von 0,8 bis 0,25 mm gefertigt werden. Die Lanzettenspitze 28 ist zu einer Spitze geschliffen, wobei die verschiedensten Schliffe möglich sind, die eine Perforation der Haut eines Testsubjektes ermöglichen. Zur Herstellung der erfindungsgemäßen Teststreifen werden eine Vielzahl von Lanzetten 26 im gegenseitigen Abstand von einer Testelementbreite mit einem thermisch schmelzbaren Elastomer (Pellethane der Firma Dow Plastics) zu einem kontinuierlichen Band 84 verbunden. Dabei ist sichergestellt, dass die einzelnen Lanzetten 26 über mindestens 1/10 ihrer Länge von dem Elastomer umfasst sind, wodurch die Kunststoffummantelung 34 ausgebildet wird. Mittig zwischen zwei Lanzetten 26 wird ein verbreiterter Bereich geformt, vergleiche 1. verbreiterter Bereich 42 und 2. verbreiterter Bereich 44, die mindestens 1/10 und maximal 1/4 der Breite des Testelementes 14 breit sind und sich über mindestens 1/10 der Länge der Lanzette 26 erstrecken. Die verbreiterten Bereiche 42 bzw. 44 weisen eine Dicke 102 auf, die 1/4 bis 5/4 der Dicke der Lanzette 26 beträgt. Zwischen der Lanzette 26 und dem 1. und 2. verbreiterten Bereich 42 und 44 wird das kontinuierliche Band 84 in seiner Dicke auf eine Dicke von maximal 200 µm (Bezugszeichen 100) reduziert. In diesem Bereich, der in verminderter Dicke 100 ausgeführt ist, werden durch Ausstanzen, Ausformen oder Einschneiden mindestens zwei Einzelstränge 38 (vergleiche Draufsicht gemäß Figur 1) der Elastomerfeder 36 gebildet, welche beidseits der Lanzette 26 Verbindungen zu den als Federwiderlager 40 dienenden 1. und 2. verbreiterten Bereichen 42 und 44 schaffen.

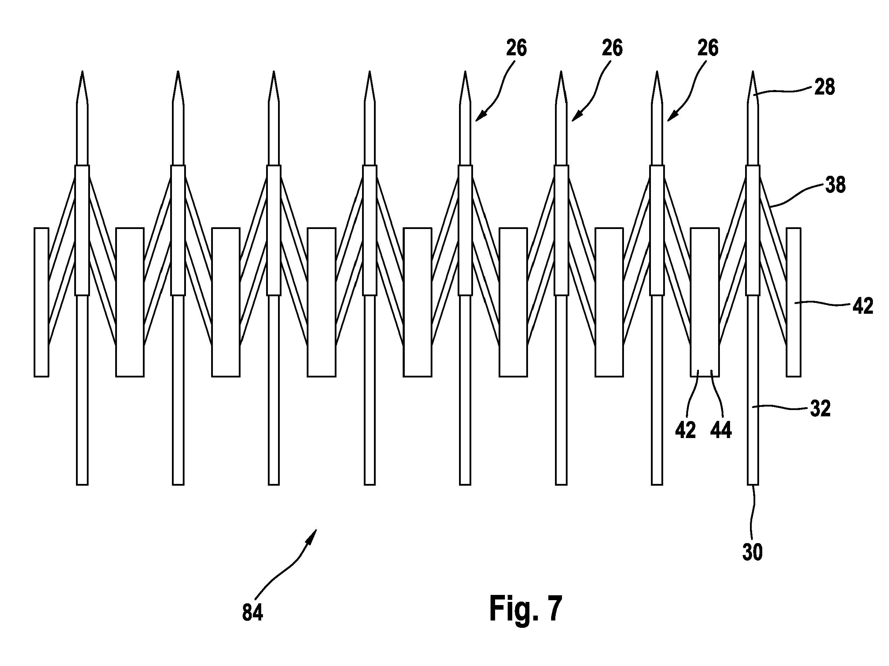

Das kontinuierliche Band 84 (vergleiche Darstellung gemäß Figur 7) wird unter Kontrolle des Abstandes der Lanzetten 26 voneinander und der Position der Lanzetten 26 auf das Sterilschutz-Folienband transportiert, aus dem die Folientasche 50 erzeugt wird. Das Sterilschutz-Folienband zeichnet sich durch eine niedrige bis moderate Reißfestigkeit, eine gute thermische Verschweißbarkeit sowie Strahlensterilisierbarkeit aus. Das Sterilschutz-Folienband kann zum Beispiel aus einem geschäumten OPP (DuPont Teijin Films) gefertigt werden. Die Breite des Sterilschutz-Folienbandes beträgt mehr als das Doppelte der Länge der einzelnen Lanzetten 26. Das Sterilschutz-Folienband ist an einer Seite mit in Figur 4 dargestellten Pilotlöchern 112 versehen. Mittels der Pilotlöcher 112, die in Figur 4 an dem Sterilschutz-Folienband ausgebildet sind, in Fig. 4 von der Oberseite 52 her dargestellt, wird eine Ausrichtung und ein exaktes Miteinanderfluchten der Einzelschichten 84, 88, 90 des Schichtaufbaus 98, wie in Fig. 3a dargestellt, relativ zueinander erzielt. Da diese aus Kunststoffmaterial gefertigt werden, ist ein Verzug derselben zueinander während der Herstellung des Lanzettensystems 10 durchaus möglich, so dass eine exakte Ausrichtung der einzelnen Schichten des Schichtaufbaus 98 des Lanzettensystems 10 über die Pilotlöcher 112 gemäß Figur 4 erreicht wird. Das kontinuierliche Band 84 mit den einzelnen, miteinander verbundenen Lanzetten 26 wird an den 1. und 2. verbreiterten Bereichen 42 bzw. 44 mit dem Sterilschutz-Folienband thermisch verbunden. Das Sterilschutz-Folienband wird über die Lanzettenspitzen 28 zu einer Folientasche 50 gefaltet. Der umgeklappte Falz 54 liegt vor den Lanzettenspitzen 28. Die entstandene Folientasche 50 wird thermisch versiegelt, so dass die einzelnen Lanzetten 26 auf einem jeden Lanzettensystem 10 einzeln verpackt und sterilisiert sind. Das erhaltene Band 84 wird aufgewickelt und einer Sterilisation, bevorzugt einer Strahlensterilisation, unterzogen.

Parallel zur Herstellung des kontinuierlichen Bandes 84 mit den Lanzetten 26, die einzeln von der Folientasche 50 umschlossen sind, werden die Testelemente 14 auf einem Trägerband 88 gefertigt. Bei diesem Band handelt es sich bevorzugt um eine Melinexfolie mit einer Dicke von 350 µm (Hersteller: Huhtamaki Deutschland, Ronsberg/Allgäu), in welcher die in Figur 5 dargestellten Pilotlöcher 112 mit einem definierten Abstand voneinander angeordnet sind, der einem ganzzahligen Vielfachen der Breite eines Testelements 14 entspricht.

Die Unterseite des Trägerbandes 88 wird mit einer Folie mit Elektrodenstrukturen 96 beschichtet, aus der nach Entfernen von überschüssigem Material die einzelnen Elektroden 66, 68, 70 samt Leiterbahnen 70, die sich in den Messbereich 64 erstrecken, geformt werden. Durch Applikation eines elektrochemischen Nachweisreagenzes, wie zum Beispiel einer Nachweispaste, können Enzym-Elektroden ausgebildet werden. Die in Figur 3a durch Bezugszeichen 90 kenntlich gemachte Spacerschicht umfasst die Kapillarkanäle 92 und ist von einer hydrophilen Deckelfolie 72 verschlossen, so dass über die derart gebildeten, von der Deckelfolie 72 verschlossenen und seitlich von der Spacerschicht 90 begrenzten Kapillarkanäle 92 Probeflüssigkeit zu den im Messbereich liegenden Elektrodenanschlüssen der Elektroden 66, 68 und 70 transportiert werden kann.

In einem Abstand von der Kapillare 62, der geringer bemessen ist als die Länge der Lanzette 26 und mit den Kapillaren 62 fluchtend, sind im Trägerband 88 rechteckige Ausnehmungen vorgesehen, die in Zusammenhang mit der Beschreibung der Figuren 1 und 2 durch die Ausnehmungskontur 48 dargestellt sind. Die Breite der Ausnehmungskontur 48 entspricht mindestens der Breite des geräteseitigen Stößels. Die Länge der Ausnehmungskontur 48 - in Stichrichtung gesehen - ergibt sich aus den Abmessungen der beteiligten Teile und der gewünschten Wegstrecke der Lanzettenspitze 28.

Während die Ausnehmungskontur 48 in den Darstellungen gemäß den Figuren 1 und 2 rechteckförmig beschaffen ist, besteht alternativ auch die Möglichkeit, die Ausnehmungskontur 48, zum Beispiel halbkreisförmig oder V-förmig auszubilden. Dadurch ist gewährleistet, dass ein Vorschubelement eines Stechantriebes ungehindert auf das stumpfe Ende 60 des Lanzettenkörpers 32 einwirken kann, um die Stichbewegung der Lanzette 26 zur Erzeugung einer Perforation in der Haut des Testsubjektes mit möglichst hoher Stichgeschwindigkeit auszuführen, was das Schmerzempfinden des Patienten beim Stich auf ein Mindestmaß reduziert.

Der Darstellung gemäß Figur 3a ist entnehmbar, dass das kontinuierliche Band 84 (vergleiche auch Darstellung gemäß Figur 7), in dem die einzelnen Lanzetten 26 seitlich voneinander beabstandet sind, von der Sterilschutzfolie beidseits als Folientasche 50 umgeben ist und über die Unterseite 82 der gebildeten Folientasche 50 mittels einer Kleberschicht 86 mit dem Folienband 88 verbunden ist. Bei der Kleberschicht 86 kann es sich sowohl um ein doppelseitiges Klebeband als auch um einen Schmelzkleber handeln. Nach der Sterilisation des kontinuierlichen Bandes 84 mit den in den Folientaschen 50 verpackten Lanzetten 26, wird mit der Kleberschicht 86 auf die der Elektrodenstruktur 96 gegenüberliegende Seite des Trägerbandes 88 der Testelemente 14 geklebt. Überstehendes Material sowohl des Sterilschutz-Folienbandes der Folientasche 50 als auch des Trägerbandes 88 und der Spacerschicht 90, in dem sich die Pilotlöcher 112 (vergleiche Darstellung gemäß der Figuren 4 oder 5) befinden, wird abgetrennt. Danach können die einzelnen Lanzettensysteme 10 vereinzelt werden und in Dosen, Magazinen oder Blisterpackungen einzeln oder zu mehreren umverpackt werden.

Die in Figur 5 dargestellte Spacerschicht 90 umfasst Teile der Kapillarkanäle 92, die in dieser eingestanzt sind. Wird ein Randbereich der Spacerschicht 90 entlang einer Abtrennkante 110 abgetrennt, so werden Öffnungen der Kapillarkanäle 92 erhalten, die innerhalb des fertig hergestellten Lanzettensystems 10 an dessen Vorderkante 16 (vergleiche Darstellung gemäß Figur 2) liegen. Die in Figur 5 schematisch dargestellte Trägerfolie 88 mit aufgeklebter Spacerschicht 90 umfasst darüber hinaus an gegenüberliegenden Rändern 108 Pilotlöcher 112, die in einem Abstand, der einem ganzzahligen Vielfachen der Breite der Testelemente 14 entspricht, voneinander beabstandet sind. Über die Pilotlöcher 112 wird eine exakte Ausrichtung der Einzelschichten 88, 90 und 72 sowie des Bandes 84 und des Sterilschutz-Folienbandes relativ zueinander erreicht.

Den Darstellungen gemäß Figuren 6 und 6.1 ist eine alternative Ausführungsvariante der in Figur 1 dargestellten Lanzette 26 zu entnehmen. Anstelle der in Figur 1 dargestellten Lanzette 26, die einen Kreisquerschnitt aufweist und als Nadel ausgebildet ist, kann die Lanzette 26 auch als Flachlanzette 114 aus einem Stahl gefertigt werden. Der Querschnitt der in Figur 5 dargestellten Flachlanzette 114 kann zum Beispiel rechteckförmig sein, gekennzeichnet in Figur 5.1 durch Bezugszeichen 118. Daneben ist es ebenso gut möglich, die Lanzette 26 in einem quadratischen Querschnitt auszubilden, was jedoch zeichnerisch nicht dargestellt ist und da es lediglich einer geometrische Abwandlung der Variante der Lanzette 26 gemäß der Figuren 6 und 6.1 entspricht.

Analog zur elastischen Aufnahme der als Nadel ausgebildeten Lanzette 26 in dem in Figur 1 dargestellten Ausführungsbeispiel des Lanzettensystems 10 kann die in Figur 6 und 6.1 dargestellte Flachlanzette 114, ebenfalls von einer Kunststoffummantelung 34 umgeben, durch mindestens zwei Stränge 38 der Elastomerfeder 36 mit den als Federwiderlager 40 dienenden 1. und 2. verbreiterten Bereichen 42 und 44 an der Umlaufkante 56 des Testelementes 14 verbunden werden. Je nach Winkellage der mindestens zwei Einzelstränge 38 der Elastomerfeder 36, die beidseits der mittig auf dem Testelement 14 aufgenommenen Lanzette 26 verlaufen, und je nach verwendeten Federeigenschaften des Materials, aus welchem das kontinuierliche Band 84 gefertigt wird, lassen sich die Geschwindigkeit der Rückzugsbewegung sowie die durch den Stechantrieb aufzubringende Stichgeschwindigkeit abzüglich der Überwindung der Elastomerdehnung auslegen, um einen für den Patienten möglichst schmerzarmen Stichvorgang zur Entnahme einer Körperflüssigkeit zu erreichen.

Der Darstellung gemäß Figur 7 ist ein kontinuierliches Band mehrerer voneinander beabstandeter Lanzetten enthaltend zu entnehmen.

Aus der Darstellung gemäß Figur 7 geht hervor, dass die einzelnen Lanzetten 26 des Lanzettensystems 10 jeweils über Stränge, Fäden oder Streifen 38 zwischen verbreiterten Bereichen 42, 44 elastisch aufgenommen sind. Der Lanzettenspitze 28 einer jeden einzelnen Lanzette 26 gegenüberliegend, befindet sich die stumpf ausgebildete Stirnfläche 30 der Lanzette 26, auf welche ein messgeräteseitiger Stößel zum Vorschub der Lanzette 26 einwirkt. Das in Figur 7 in der Draufsicht dargestellte kontinuierliche Band 84 mit einzelnen Lanzetten 26 ist in der Schnittdarstellung des Lanzettensystems 10 gemäß Figur 3 im Querschnitt dargestellt.

### Bezugszeichenliste

- 10: Lanzettensystem
- 12: Vorderseite
- 14: Testelement
- 16: Vorderkante
- 18: Hinterkante
- 20: 1. Längsseite
- 22: 2. Längsseite
- 24: Fläche Testelemente
- 26: Lanzette
- 28: Lanzettenspitze
- 30: Stumpfes Ende Lanzette

- 32: Lanzettenkörper
- 34: Kunststoffummantelung
- 36: Elastomerfeder
- 38: Strang (Faden, Streifen)
- 40: Federwiderlager Testelement
- 42: 1. verbreiterter Bereich (42a hintere Schmalkante, 42b vordere Schmalkante)
- 44: 2. verbreiterter Bereich (44a hintere Schmalkante, 44b vordere Schmalkante)
- 46: Einschnitt, Ausstanzung
- 48: Ausnehmungskontur
- 50: Sterilschutz-Folienband (Folientasche)
- 52: Oberseite Folientasche
- 54: Falz Folientasche
- 56: Umlaufkante
- 58: Rückseite Testelement 14
- 60:
- 62: Kapillareingang
- 64: Messbereich
- 66: 1. Elektrode
- 68: 2. Elektrode
- 70: 3. Elektrode
- 71: Leiterbahn
- 72: Deckelfolie
- 74:
- 76:
- 78:
- 80: Trennkante
- 82: Unterseite Folientasche
- 84: Kontinuierliches Band mit Lanzetten 26
- 86: Kleberschicht (Schmelzkleber)
- 88: Trägerband
- 90: Spacerschicht
- 92: Kapillare
- 96: Folie mit Elektrodenstruktur
- 98: Schichtaufbau
- 100: Dicke Elastomerfeder 36
- 102: Dicke Elastomerfeder Widerlager 40
- 104:
- 106:
- 108: Rand
- 110: Abtrennkante Randbereich
- 112: Pilotlöcher
- 114: Flachlanzette
- 116: Lanzettenspitze
- 118: Flachlanzettenquerschnitt

## Patentansprüche

1. Lanzettensystem (10) zum Nachweis eines Analyten in einer Körperflüssigkeit, ein Testelement (14) und eine relativ zum Testelement (14) bewegbare Lanzette (26) umfassend, die am Testelement (14) aufgenommen ist, **dadurch gekennzeichnet**, **das** die Lanzette (26) über eine Elastomerfeder (36) elastisch mit dem Testelement (14) verbunden ist.

2. Lanzettensystem (10) gemäß Anspruch 1, **dadurch gekennzeichnet, dass** die Lanzette (26) parallel zur Längsachse des Testelementes (14) ausgerichtet ist.

3. Lanzettensystem (10) gemäß Anspruch 1, **dadurch gekennzeichnet, dass** die Elastomerfeder (36) mindestens einen federnden Bereich (38) aufweist, der sich beidseits der Lanzette (26) erstreckt.

4. Lanzettensystem (10) gemäß Anspruch 3, **dadurch gekennzeichnet, dass** der mindestens eine federnde Bereich (38) in Form eines Stranges, oder eines Fadens oder eines Streifens ausgebildet ist.

5. Lanzettensystem (10) gemäß Anspruch 3, **dadurch gekennzeichnet, dass** sich der mindestens eine federnde Bereich (38) vom Lanzettenkörper (32) der Lanzette (26) zu einer 1. Längsseite (20) und einer 2. Längsseite (22) des Testelementes (14) erstreckt.

6. Lanzettensystem (10) gemäß Anspruch 3, **dadurch gekennzeichnet, dass** die Elastomerfeder (36) aus einem gummielastischen Material gefertigt ist.

7. Lanzettensystem (10) gemäß Anspruch 3, **dadurch gekennzeichnet, dass** die Elastomerfeder (36) in dem mindestens einen federnden Bereich (38) eine reduzierte Dicke (100) von maximal 200 µm aufweist.

8. Lanzettensystem (10) gemäß Anspruch 5, **dadurch gekennzeichnet, dass** an der 1. Längsseite (20) und an der 2. Längsseite (22) des Testelementes (14) jeweils 1. und 2. verbreitere Bereiche (42, 44), die als Federwiderlager (40) der Elastomerfeder (36) dienen, ausgeführt sind.

9. Lanzettensystem (10) gemäß Anspruch 8, **dadurch gekennzeichnet, dass** die 1. und 2. verbreiterten Bereiche (42, 44) in einer Dicke (102) ausgeführt sind, die 1/4 bis 5/4 der Dicke der Lanzette (26) entspricht.

10. Lanzettensystem (10) gemäß Anspruch 4, **dadurch gekennzeichnet, dass** der mindestens eine federnde Bereich (38) der Elastomerfeder (36) durch Einschnitte, Ausstanzungen oder Ausformungen (46) im Elastomermaterial begrenzt ist.

11. Lanzettensystem (10) gemäß Anspruch 1, **dadurch gekennzeichnet, dass** die Lanzette (26) eine Lanzettenspitze (28) aufweist, die mit einer auf einer Vorderseite (12) oder auf einer Rückseite (60) des Testelementes (14) verlaufenden Kapillare (92) fluchtet, die an einer Vorderkante (16) des Lanzettensystems (10) mündet.

12. Lanzettensystem (10) gemäß Anspruch 8, **dadurch gekennzeichnet, dass** die 1. und 2. verbreiterten Bereiche (42, 44) des Testelementes (14) an der 1. und 2. Längsseite (20, 22) des Testelementes (14) in Bezug auf die Längsachse des Testelementes (14) versetzt zu einer Kunststoffummantelung (34) der Lanzette (26) angeordnet sind.

13. Lanzettensystem (10) gemäß Anspruch 3, **dadurch gekennzeichnet, dass** der mindestens eine federnde Bereich (38) in Bezug auf die Längsachse des Testelementes (14) gepfeilt auf dessen Vorderkante (16) gerichtet verläuft.

14. Lanzettensystem (10) gemäß Anspruch 1, **dadurch gekennzeichnet, dass** dieses von einer Folientasche (50; 52, 82) aus Sterilschutz-Folienband sterilisiert versiegelt ist.

15. Lanzettensystem (10) gemäß Anspruch 14, **dadurch gekennzeichnet, dass** die Folientasche (50; 52, 82) entlang einer Umlaufkante (56) des Testelementes (14) mit diesem verbunden ist.

16. Lanzettensystem (10) gemäß Anspruch 14, **dadurch gekennzeichnet, dass** die Folientasche (50; 52, 82) an der Vorderkante (16) des Lanzettensystems (10) mit einem Falz (54) versehen ist.

17. Lanzettensystem (10) gemäß Anspruch 1, **dadurch gekennzeichnet, dass** in einer Fläche (24) des Testelementes (14) eine Ausnehmungskontur (48) geschaffen ist, innerhalb der ein stumpfes Ende (30) der Lanzette (26) freilegbar ist.

18. Lanzettensystem (10) gemäß Anspruch 1, **dadurch gekennzeichnet, dass** die Lanzette (26) nadelförmig mit einem kreisrunden Querschnitt ausgeführt ist oder als Flachlanzette (114) ausgeführt ist, die in einen im Wesentlichen rechteckigen Querschnitt aufweist.

19. Lanzettensystem (10) gemäß Anspruch 1, **dadurch gekennzeichnet, dass** die Länge der Lanzette (26) kleiner ist als die Längserstreckung des Testelementes (14), mit dem die Lanzette (26) elastisch verbunden ist.

20. Verfahren zur Herstellung eines Lanzettensystems (10), insbesondere zum Nachweis eines Analyten in einer Körperflüssigkeit gemäß einem oder mehreren der vorhergehenden Ansprüche mit nachfolgenden Verfahrensschritten:
a) Aus einer Vielzahl voneinander beabstandeter Lanzetten (26) und einem thermisch schmelzbaren Elastomer wird ein kontinuierliches Band (84) geformt,
b) in dem kontinuierlichen Band (84) wird durch Einschnitte, Ausformungen oder Ausstanzungen (46) mindestens ein federnder Bereich (38) einer Elastomerfeder (36) erzeugt,
c) das kontinuierliche Band (84) wird nach Durchführung des Verfahrensschrittes b) auf ein Sterilschutz-Folienband (50) aufgebracht, aus welchem eine das Lanzettensystem (10) steril haltende Folientasche (50; 52, 82) erzeugt wird und
d) das versiegelte Lanzettensystem (10) wird mit einer das Testelement (14) bildenden Spacerschicht (90) gefügt, in welchem die Kapillare (92) verläuft und ein Messbereich (64) zur Bestimmung eines Analyten in einer Körperflüssigkeit ausgebildet ist.

21. Verfahren gemäß dem vorhergehenden Anspruch, **dadurch gekennzeichnet, dass** die Lanzetten (26) gemäß Verfahrensschritt a) in einem Abstand von einer Testelementbreite zueinander angeordnet sind.

22. Verfahren gemäß Anspruch 20, **dadurch gekennzeichnet, dass** die Lanzetten (26) gemäß Verfahrensschritt a) mit einer Kunststoffummantelung (34) überzogen werden, die sich in einer Länge von mindestens 1/20 der Länge des Lanzettenkörpers (32) erstreckt.

23. Verfahren gemäß Anspruch 20, **dadurch gekennzeichnet, dass** im kontinuierlichen Band (84) zwischen zwei Lanzetten (26) 1. und 2. verbreiterte Bereiche (42, 44) in einer Breite geformt werden, die mindestens 1/20 und maximal 1/4 der Breite des Testelementes (14) beträgt.

24. Verfahren gemäß dem vorhergehenden Anspruch, **dadurch gekennzeichnet, dass** zwischen der Lanzette (26) und dem 1. und 2. verbreiterten Bereich (42, 44) des kontinuierlichen Bandes (84) dieses in einer reduzierten Dicke (100) von maximal 200 µm ausgebildet wird.

25. Verfahren gemäß Anspruch 20, **dadurch gekennzeichnet, dass** gemäß Verfahrensschritt c) das Lanzettensystem (10) auf das Sterilschutz-Folienband (50) aufgebracht wird, dessen Breite mindestens der doppelten Länge der Lanzette (26) entspricht.

26. Verfahren gemäß Anspruch 20, **dadurch gekennzeichnet, dass** gemäß Verfahrensschritt c) das kontinuierliche Band (84) und das Sterilschutz-Folienband (50) thermisch gefügt werden.

27. Verfahren gemäß Anspruch 20, **dadurch gekennzeichnet, dass** nach Verfahrensschritt c) das kontinuierliche Band (84) in der Folientasche (50; 52, 82) thermisch versiegelt und danach einem Sterilisationsverfahren, bevorzugt einer Strahlensterilisation, unterzogen wird.

28. Verfahren gemäß Anspruch 20, **dadurch gekennzeichnet, dass** das Lanzettensystem (10) als ein Schichtaufbau (98), eine Deckelfolie (72), eine Spacerschicht (90) mit darin eingebrachten Kapillaren (92), ein Trägerband (88), eine Kleberschicht (86) und das in einer Folientasche (50; 52, 82) versiegelte kontinuierliche Band (84) aufweisend, hergestellt wird.

29. Verfahren gemäß dem vorhergehenden Anspruch, **dadurch gekennzeichnet, dass** die einzelnen Schichten (90, 88, 72) und das kontinuierliche Band (84) des Lanzettensystems (10) während des thermischen Fügens über in Randbereichen der Schicht (90, 88, 84) ausgebildete Pilotlöcher (112) zueinander ausgerichtet und positioniert werden.
